(19) Europäisches Patentamt / European Patent Office / Office européen des brevets

(11) **EP 4 066 737 A1**

(12) **EUROPEAN PATENT APPLICATION**
published in accordance with Art. 153(4) EPC

(43) Date of publication:
**05.10.2022 Bulletin 2022/40**

(21) Application number: **20893172.5**

(22) Date of filing: **16.11.2020**

(51) International Patent Classification (IPC):
**A61B 5/053** (2021.01)    **A61B 5/00** (2006.01)

(52) Cooperative Patent Classification (CPC):
**A61B 5/00; A61B 5/053**

(86) International application number:
**PCT/JP2020/042618**

(87) International publication number:
**WO 2021/106654 (03.06.2021 Gazette 2021/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.11.2019 JP 2019213449**

(71) Applicant: **Skinos Co., Ltd.
Ueda-shi, Nagano, 386-0017 (JP)**

(72) Inventors:
• **MOMOSE, Hideya
  Ueda-shi, Nagano 386-0017 (JP)**
• **OHHASHI, Toshio
  Matsumoto-shi, Nagano 390-0812 (JP)**
• **SAKAGUCHI, Masao
  Tomi-shi, Nagano 389-0505 (JP)**

(74) Representative: **Gille Hrabal
Partnerschaftsgesellschaft mbB
Patentanwälte
Brucknerstraße 20
40593 Düsseldorf (DE)**

(54) **TOTAL BODY WATER CONTENT EVALUATION SYSTEM**

(57)    A total body water content evaluation system including a calculation device that calculates a total body moisture loss amount per unit time from a local perspiration amount (mg/cm$^2$/min) obtained using a perspiration meter and a following formula (1),

$$\text{total body moisture loss amount per unit time (mg/min)} = \text{local perspiration amount (mg/cm}^2\text{/min)} \times \text{correction coefficient for each measurement site} \times \text{body surface area (cm}^2) \ldots (1).$$

EP 4 066 737 A1

**Description**

Technical Field

The present invention relates to a total body water content evaluation system.

Background Art

A human body includes a bodily fluid (blood, lymph fluids, digestive juices), which is approximately 60% of the body weight.

The bodily fluid has a function of delivering oxygen captured by respiration and nutrients obtained from food and the like into the body. When a state where the bodily fluid is less than normal (hereinafter, referred to as dehydration) is uncontrolled, the oxygen and the nutrients necessary for normally working the blood vessels, the internal organs, the brain, and the like are insufficient, which may cause an unexpected disease.

In particular, aged persons easily fall into the dehydration because the amount of bodily fluid (hereinafter, also refereed to as total body water content) is lowered to about 50% of the body weight, and a visceral function and a sensory function are also lowered. In recent years, with the graying of the society, concern about the prevention of dehydration in aged persons is increasing.

Moreover, in sporting, it is considered that a decrease in the performance level begins about when the total body water content is lowered to about 1% of the body weight. In recent years, with the spread of the sports science, an interest in suitable water replacement in sporting is increasing.

As for a body moisture meter that measures the moisture in a living body of a subject, a technology in which at least one selected from a body weight, a hematocrit value, a serum total protein, a serum sodium concentration, and a plasma osmotic pressure is used as a parameter, a reference value and a state value of the parameter are input to the body moisture meter, and a body status quantity related to the water content in the body is calculated using the input parameter is disclosed (PTL 1).

However, the body moisture meter in PTL 1 calculates a body status quantity using a parameter measured in advance, and is thus not suitable for use of evaluating a moisture loss amount of the total body in real time, for example, during sports, because a device for parameter measurement needs to be prepared independent of the body moisture meter and the parameter needs to be measured in advance.

Citation List

Patent Literature

PTL 1: Japanese Patent No. 6117101

Summary of Invention

Technical Problem

The present invention has been made in view of such circumstances, and aims to provide a total body water content evaluation system capable of evaluating a moisture loss amount of a total body in real time with a compact device configuration.

Solution to Problem

The present invention provides the following [1] to [9].

[1] A total body water content evaluation system including a calculation device that calculates a total body moisture loss amount per unit time from a local perspiration amount ($mg/cm^2/min$) obtained using a perspiration meter and a following formula (1).

$$\text{Total body moisture loss amount per unit time (mg/min)} = \text{local perspiration amount (mg/cm}^2\text{/min)} \times \text{correction coefficient for each measurement site} \times \text{body surface area (cm}^2) \ldots (1)$$

[2] The total body water content evaluation system according to [1], in which the perspiration meter is any one selected from a group consisting of following (A) to (D):

(A) a perspiration meter including at least one or more types of sensors that measure a water content to be generated from a skin surface, and one or more evaporation promoting mechanisms that promote evaporation on the skin surface;
(B) a perspiration meter including two or more types of sensors that measure the water content to be generated from the skin surface;
(C) a perspiration meter including one or more types of sensors that measure the water content to be generated from the skin surface, a moisture-proof mechanism, and a discharge unit of a liquid; and
(D) a perspiration meter including: a housing capsule including an opening portion to be attached to the skin surface, the housing capsule including an air suction hole for sucking natural air into an inside of the housing capsule, a mixture chamber that communicates with the opening portion to diffuse sweat on the skin surface, and in which the diffused sweat and the natural air are mixed and are made to be mixed air, and an air discharge hole for discharging the mixed air from the mixture chamber; a first humidity sensor for measuring humidity of the natural air; and a second humidity sensor for measuring humidity of the mixed air.

[3] The total body water content evaluation system according to [1] or [2], further including an input device having an input function of a measurement start instruction and a measurement cycle (min), in which the calculation device has a function of calculating a cumulated moisture loss amount (mg) subsequent to the measurement start instruction using the total body moisture loss amount per unit time (mg/min) and the measurement cycle (min).

[4] The total body water content evaluation system according to [3], in which the input device has a function of inputting body weight data, and the calculation device has a function of calculating a water decrease rate (%) from the cumulated moisture loss amount (mg) and the body weight data.

[5] The total body water content evaluation system according to [4], further including a storage device that has stored therein a level display and/or a warning display in accordance with the water decrease rate (%).

[6] The total body water content evaluation system according to [5], further including a display device that displays the level display and/or the warning display.

[7] The total body water content evaluation system according to [6], in which the calculation device selects a level display and/or a warning display in accordance with the water decrease rate (%) from the storage device, and displays the level display and/or the warning display on the display device.

[8] The total body water content evaluation system according to [3], further including a display device that gives notice of an increase in the cumulated moisture loss amount (mg).

[9] The total body water content evaluation system according to [8], in which the calculation device calculates an increase rate of the local perspiration amount ($mg/cm^2/min$), and gives the notice when the increase rate decreases.

Advantageous Effects of Invention

The present invention can provide a total body water content evaluation system capable of evaluating the water content of a total body in real time with a compact device configuration.

Brief Description of Drawings

Fig. 1 is a schematic explanatory diagram of a total body water content evaluation system according to one embodiment of the present invention. A schematic explanatory diagram of a perspiration meter in an embodiment (D) is included.

Fig. 2 is a device configuration diagram of the total body water content evaluation system according to one embodiment of the present invention.

Fig. 3A is a schematic explanatory diagram of a perspiration meter in an embodiment (A).

Fig. 3B is a schematic explanatory diagram of a perspiration meter in the embodiment (A).

Fig. 4 is a schematic explanatory diagram of a perspiration meter in an embodiment (B).

Fig. 5 is a schematic explanatory diagram of a perspiration meter in an embodiment (C).

Fig. 6 is an explanatory diagram in an example, and is a diagram illustrating a measurement result of the local perspiration amount ($mg/cm^2/min$) by the perspiration meter, and body weight measurement timing.

Fig. 7 is an explanatory diagram in the example, and illustrates comparison data between the cumulated moisture loss amount (g) subsequent to a measurement start instruction, calculated by the total body water content evaluation system and a body weight reduction amount (g).

Description of Embodiments

Hereinafter, a total body water content evaluation system in one embodiment of the present invention will be described in details. Further, the present invention is not limited to the following embodiment.

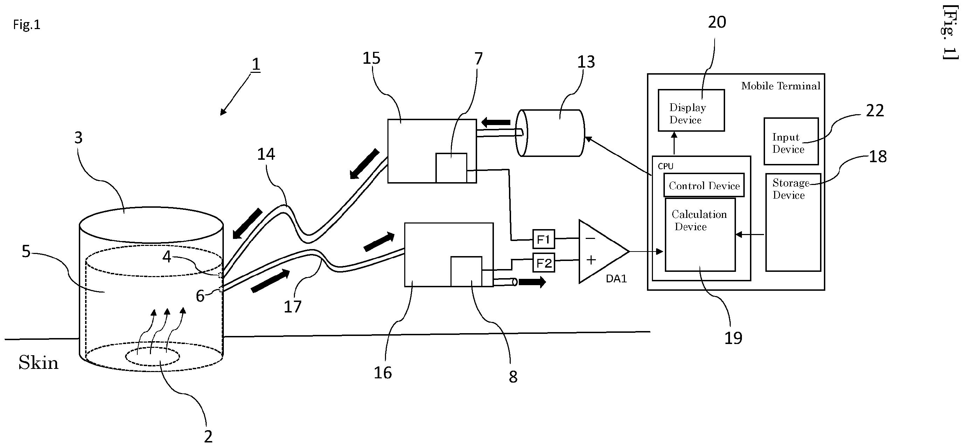

A total body water content evaluation system in the present embodiment is provided with a perspiration meter 1 and a calculation device 19, as illustrated in Fig. 1. The calculation device 19 calculates a total body moisture loss amount per unit time (mg/min) from a local perspiration amount (mg/cm$^2$/min) obtained using the perspiration meter 1 and a following formula (1).

$$\text{Total body moisture loss amount per unit time (mg/min)} = \text{local}$$
$$\text{perspiration amount (mg/cm}^2\text{/min)} \times \text{correction coefficient for each measurement}$$
$$\text{site} \times \text{body surface area (cm}^2) \ldots (1)$$

The "correction coefficient for each measurement site" in the formula (1) is a coefficient corresponding to the easiness of the perspiration in a measurement site to which the perspiration meter 1 is attached, becomes a small value when a target is the forehead, the chest, the back, or the like having a large perspiration amount, and becomes a large value when a target is the limbs or the like having a small perspiration amount, for example. In a case where a measurement site to be targeted by the total body water content evaluation system is determined to one location, the "correction coefficient for each measurement site" may be a fixed value, but the total body water content evaluation system may preferably have a function of correcting an individual difference in order to improve the calculation accuracy of correcting the total body moisture loss amount per unit time. Specifically, in accordance with a following formula (2), it is preferable to compare body weight reduction in exercising during certain time with a cumulated value of local perspiration amounts in a measurement site during the certain time, and to obtain a correction coefficient for each measurement site to which a individual difference is added. When a correction coefficient in the following formula (2) is obtained, no water replacement is performed during exercise.

$$\text{Correction coefficient for each measurement site to which individual}$$
$$\text{difference is added} = (\text{body weight [kg] before exercise]} - \text{body weight [kg] after}$$
$$\text{exercise}) / \text{body surface area [cm}^2] / \text{cumulation of local perspiration amounts}$$
$$[\text{mg/cm}^2] \ldots (2)$$

The specific mode of the calculation device 19 is not specially limited, and for example, a CPU of a mobile terminal can be used.

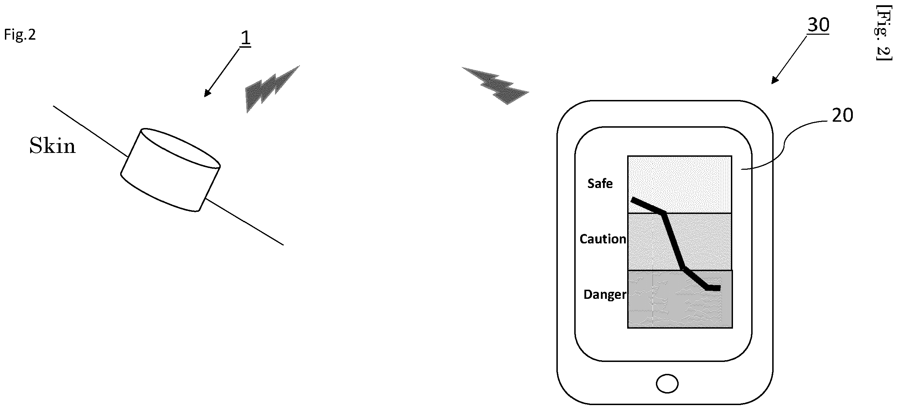

In a case where the CPU of the mobile terminal is used as the calculation device 19, as illustrated in Fig. 2, a calculation result by the calculation device can be displayed on a screen of a mobile terminal 30, which serves as a display device 20.

[Perspiration Meter]

The perspiration meter 1 is not specially limited, and preferably includes a perspiration meter that measures the amount of moisture (hereinafter, water content) that is generated from a surface of a skin (hereinafter, referred to as skin surface), a perspiration meter that electrically measures the impedance of a skin, a perspiration meter that can measure a temporal change in the local perspiration amount (local perspiration amount) from a sensor attached to a portion of the human body, or the like. In any case, a compact device configuration is specially preferable. The perspiration meter 1 is preferably of a wrist band type, for example, so as to be suitable when being attached in sporting.

When sweat is discharged from the skin as a liquid, a part thereof evaporates, and a part thereof remains on the skin as a liquid. The former is called as effective perspiration and the latter is called invalid perspiration, and the ratio therebetween changes depending on an environment condition on the skin surface. For example, when the humidity is low, the sweat easily evaporates to increase the effective perspiration. The perspiration amount is a total sum of the water vapor content evaporated by the effective perspiration and the water content remained on the skin as a liquid by the invalid perspiration.

A perspiration meter in an embodiment (A) is provided with at least one or more types of sensors that measure the water content to be generated from a skin surface, and one or more evaporation promoting mechanisms that promote the evaporation on the skin surface.

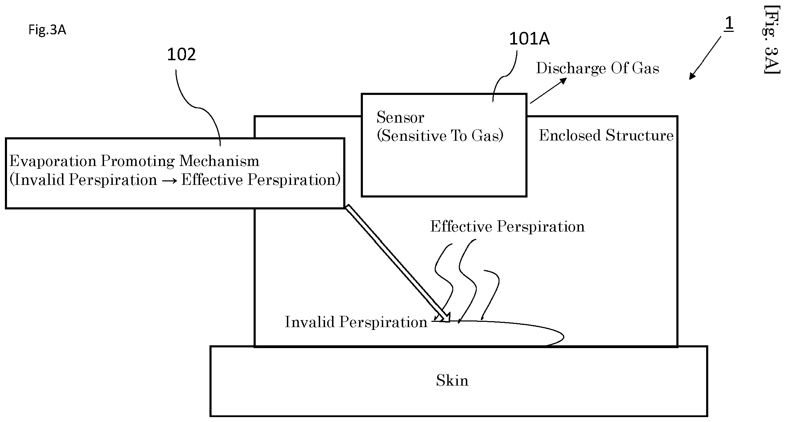

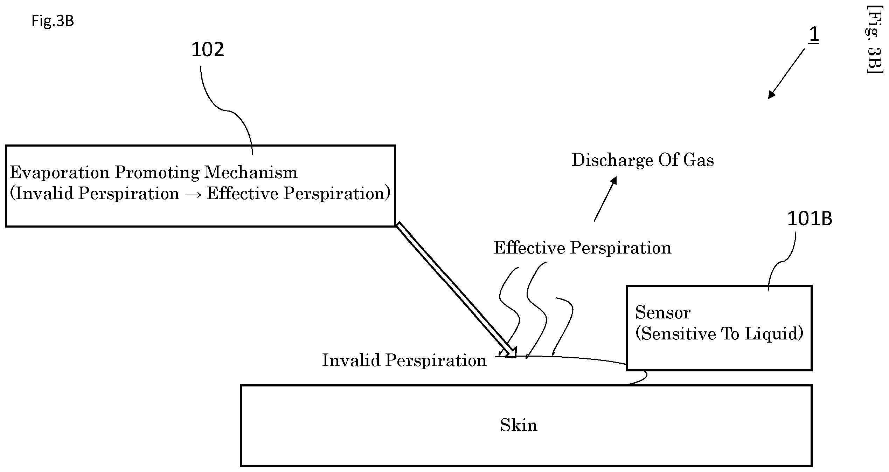

The perspiration meter in the present embodiment is preferably configured to include, as illustrated in Fig. 3A, a sensor 101A that is sensitive to the gas and can detect the effective perspiration with high sensitivity, and one or more evaporation promoting mechanisms 102 that encourage the evaporation on the skin surface, or as illustrated in Fig. 3B, a sensor 101B that is sensitive to the liquid and can detect the invalid perspiration with high sensitivity, and the one or more evaporation promoting mechanisms 102 that encourage the evaporation on the skin surface.

<Sensor>

Examples of the sensor can include a humidity sensor, an impedance sensor, an optical sensor, a mass sensor, a volume sensor, a color sensor, a heat/temperature sensor, and a camera. In addition, a component sensor for a component (a mineral and an electrolyte such as potassium, magnesium, zinc, iron, and bicarbonate ions, a lactic acid, urea, or the like) contained in the sweat may be employed.

The humidity sensor uses a rise in the humidity on the skin surface due to the generation of the moisture. The impedance sensor uses a change in the impedance of the skin due to the generation of the moisture. The optical sensor uses, for example, a light emitting element, such as 1.45 $\mu$m, that is characteristically absorbed in water, and uses the attenuation amount of the light. The optical sensor is effective for not only the liquid but also for the water vapor. The mass sensor measures and uses the weight of water. The volume sensor measures and uses the volume of water. The camera captures water droplets to be output from the skin as a video, and uses the number of the water droplets. The color sensor is effective in a case where a color-developing substance that reacts with water is applied to the skin, and captures the change in the color. The heat/temperature sensor captures the movement of the temperature and the heat that change due to the vaporization heat being deprived when the moisture evaporates.

Among the respective sensors, as the sensor 101A capable of detecting the effective perspiration with high sensitivity, the humidity sensor, the optical sensor, the heat/temperature sensor, or the like is suitable, and as the sensor 101B capable of detecting the invalid perspiration with high sensitivity, the impedance sensor, the mass sensor, the volume sensor, the camera, or the like is suitable.

<Evaporation Promoting Mechanism>

Examples of the evaporation promoting mechanism can include a ventilation device such as a compressor, a dehumidifier system such as a silica gel that lowers the humidity of the skin surface, and a heater that raises the temperature on the skin surface and lowers the relative humidity.

With the perspiration meter in the present embodiment that is provided with the evaporation promoting mechanism, sweat after having been detected by the sensor is discharged without remaining on the skin or in a sensor detector, so that it is possible to easily obtain a time change in the perspiration amount (local perspiration amount ($mg/cm^2/min$)) with a compact device configuration.

A perspiration meter in an embodiment (B) is provided with two or more types of sensors that measure the water content to be generated from the skin surface.

As the two or more types of the sensors, two or more types selected from the group of the sensors exemplified in the embodiment (A) can be used.

As for the two or more types of the sensors, the sensor 101A capable of detecting the effective perspiration with high sensitivity and the sensor 101B capable of detecting the invalid perspiration with high sensitivity are preferably used in combination.

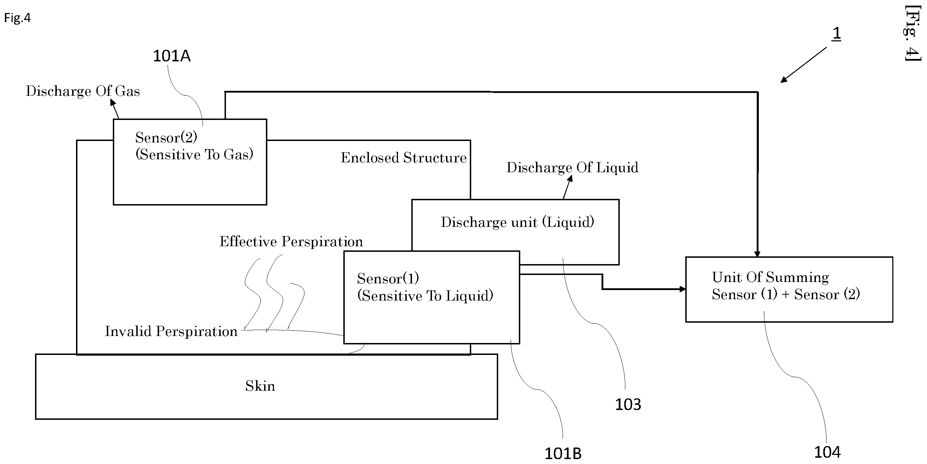

The perspiration meter in the present embodiment is preferably provided with, as illustrated in Fig. 4, in addition to the two or more types of the sensors (101A, 101B), a discharge unit 103, and a unit 104 that sums perspiration amounts to be obtained from the two or more types of the sensors.

In order to measure the perspiration amount easily and high-accurately, it is preferable to measure the entire liquid instantly made to gas, and discharge the gas thereafter, or to measure the liquid, and discharge by instantly evaporating the liquid thereafter.

In addition to the two or more types of the sensors, the discharge unit and the unit that sums perspiration amounts to be obtained from the two or more types of the sensors are provided, so that the sweat after having been detected by the sensor is discharged without remaining on the skin and in the sensor detector, and it is possible to easily obtain a time change in the perspiration amount (local perspiration amount ($mg/cm^2/min$)) with a compact device configuration.

A perspiration meter in an embodiment (C) is provided with one or more types of sensors that measure the water content to be generated from the skin surface, a moisture-proof mechanism, and a discharge unit of a liquid.

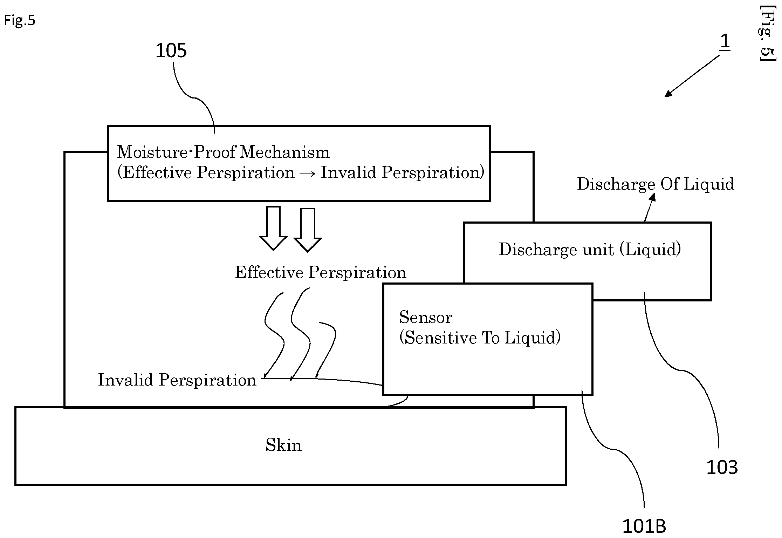

The perspiration meter in the present embodiment is preferably provided with, as illustrated in Fig. 5, the sensor 101B capable of detecting the invalid perspiration with high sensitivity, a moisture-proof mechanism 105, and the discharge unit 103 of a liquid.

The moisture-proof mechanism is provided to enable the perspiration amount of sweat as a liquid to be measured.

In a case where the moisture-proof mechanism is simply provided, a large storage structure for storing sweat is needed, however, as illustrated in Fig. 5, the moisture-proof mechanism and the discharge unit of a liquid are provided, so that it is possible to easily obtain, a time change in the perspiration amount (local perspiration amount ($mg/cm^2/min$)) with a compact device configuration.

A perspiration meter in an embodiment (D) is provided with a housing capsule 3 including an opening portion 2 to be attached to a skin surface, as illustrated in Fig. 1, the housing capsule 3 including an air suction hole 4 for sucking natural air into an inside of the housing capsule 3, a mixture chamber 5 that communicates with the opening portion 2 to diffuse sweat on the skin surface, and in which the diffused sweat and the natural air are mixed and are made to be mixed air, and an air discharge hole 6 for discharging the mixed air from the mixture chamber 5, a first humidity sensor 7 for measuring humidity of the natural air, and a second humidity sensor 8 for measuring humidity of the mixed air.

<Housing Capsule 3>

The form of the housing capsule 3 is not specially limited, and can be formed as a substantially tubular shape (as one example, a substantially cylindrical shape) in which an upper end thereof is closed, for example.

The material for the housing capsule 3 is not specially limited either, and can be formed of a synthetic resin, for example.

The air suction hole 4 for sucking natural air into the inside of the housing capsule 3 has a diameter smaller (size smaller) than an inner diameter of the tubular housing capsule 3.

The housing capsule 3 includes the opening portion 2 to be attached (adhered) to a skin surface at a lower end (at a side of the skin surface).

The opening area of the opening portion 2 is not limited, and can be 1 $cm^2$, for example. In a case where the unit of the perspiration amount on the skin surface is expressed as $mg/cm^2min$, when the opening area in the opening portion 2 is 1 $cm^2$, the measurement value of the perspiration amount can be used as it is without being divided by the area and converted.

The adhesion of the housing capsule 3 to the skin surface can be performed by applying a double-sided adhesive tape, a bonding agent, or an adhesive, for example, to a peripheral part of the opening portion 2.

The housing capsule 3 includes the mixture chamber 5 that communicates with the opening portion 2, at an inner side thereof.

The mixture chamber 5 has a function as a space in which sweat on the skin surface in a state where the mixture chamber 5 is attached to the skin surface is diffused, and the diffused sweat and the natural air are mixed.

The air discharge hole 6 for discharging the mixed air from the mixture chamber 5 has a diameter smaller (size smaller) than the inner diameter of the tubular housing capsule 3.

<Air Blowing Unit 13>

The perspiration meter 1 is preferably provided with an air blowing unit 13 that blows the natural air or a suction machine that sucks the mixed air.

The air blowing unit 13 and the suction machine may be disposed in the inside of the housing capsule 3, and may be disposed in an outside of the housing capsule 3.

As the embodiment illustrated in Fig. 1, in a case where the air blowing unit 13 is present in the outside of the housing capsule, for example, a compressor can be used as the air blowing unit 13. The compressor sucks the natural air, and then sends out the natural air at a constant flow rate. The natural air sent out from the compressor is supplied to the mixture chamber 5 in the housing capsule 3 through a flexible pipe 14.

In a case where the air blowing unit 13 is present in the inside of the housing capsule 3, the air blowing unit 13 is preferably disposed at an upstream of the mixture chamber 5. The air blowing unit 13 disposed at the upstream of the mixture chamber 5 sucks the natural air from the air suction hole 4, and blows the air to the mixture chamber 5. For example, an electric air fan, an air pump, or an air blower can be used as the air blowing unit 13.

<First Humidity Sensor 7>

The first humidity sensor 7 has a function of measuring humidity of the natural air to be sucked from the air suction hole 4.

In the embodiment illustrated in Fig. 1, the first humidity sensor 7 is disposed in an inside of a box 15 that is present in the outside of the housing capsule 3.

In the embodiment illustrated in Fig. 1, the first humidity sensor 7 measures absolute humidity of natural air sent out from the compressor serving as the air blowing unit 13, and supplied to the inside of the box 15.

In place of the first humidity sensor 7 that measures the absolute humidity, by disposing a relative humidity sensor and a temperature sensor in the inside of the box 15, the absolute humidity of natural air flowing in the inside of the box 15 can also be obtained. The temperature sensor and the humidity sensor may be integrally formed and be used.

<Second Humidity Sensor 8>

The second humidity sensor 8 has a function of measuring humidity of the mixed air.

In the embodiment illustrated in Fig. 1, the second humidity sensor 8 is disposed in an inside of a box 16 that is present in the outside of the housing capsule 3.

In the embodiment illustrated in Fig. 1, the second humidity sensor 8 measures absolute humidity discharged from the housing capsule 3 and supplied to the inside of the box 16.

In place of the second humidity sensor 8 that measures the absolute humidity, by disposing a relative humidity sensor and a temperature sensor in the inside of the box 16, the absolute humidity of mixed air flowing in the inside of the box 16 can also be obtained. The temperature sensor and the humidity sensor may be integrally formed and be used.

[Calculation Device]

The calculation device 19 calculates a total body moisture loss amount per unit time from a local perspiration amount (mg/cm$^2$/min) obtained using the perspiration meter 1 and the following formula (1).

$$\text{Total body moisture loss amount per unit time (mg/min)} = \text{local perspiration amount (mg/cm}^2\text{/min)} \times \text{correction coefficient for each measurement site} \times \text{body surface area (cm}^2) \dots (1)$$

The calculation device 19 can also further have function of calculating a cumulated moisture loss amount (mg) subsequent to a measurement start instruction, from a measurement cycle (min) input in advance, elapsed time subsequent to the measurement start instruction, and the total body moisture loss amount per unit time (mg/min). The function is included to allow a total body moisture loss amount in real time to be evaluated during sports, for example.

The calculation device 19 may also further have a function of calculating a water decrease rate (%), from body weight data input in advance and the cumulated moisture loss amount (mg).

[Storage Device]

The total body water content evaluation system of the present invention can be provided with a storage device 18 that stores therein dehydration prevention data suitable for use in order to prevent a dehydration symptom, and performance reduction prevention data suitable for use in order to prevent the performance reduction in sporting, for example.

As for the dehydration prevention data suitable for use in order to prevent the dehydration symptom, for example, those indicated in a following table 1 can be used. As for the performance reduction prevention data, for example, those indicated in the following table 2 can be used, respectively.

[Table 1]

| Moisture Decrease Rate (%) ("Amount Of Dewater replacement (g) / Initial Body Weight (g)" * 100) | Main Symptom Of Dewater replacement | Level display | Warning Display |
|---|---|---|---|
| less than 2% | None | Safe 1 | None |
| 2~3% | Thirst | Caution 1 | Please promptly receive water replacement of • ml. |

(continued)

| Moisture Decrease Rate (%) ("Amount Of Dewater replacement (g) / Initial Body Weight (g)" * 100) | Main Symptom Of Dewater replacement | Level display | Warning Display |
|---|---|---|---|
| 3~4% | Strong thirst, dazed, loss of appetite | Caution 2 | Please promptly receive water replacement of • ml. |
| 4~5% | Flush of skin, irritated, rise in body temperature, exhaustion, decrement and concentration of urine volume | Caution 3 | Please stop activity, and promptly receive water replacement of • ml. |
| 5~8% | Headache, feel oppressed by heat | Caution 4 | Please stop activity, and promptly receive water replacement of • ml. |
| 8~10% | Body rocking, convulsion | Danger 1 | Please receive medical examination of a doctor promptly. |
| 20% or more | Anuria, death | Danger 2 | Please receive medical examination of a doctor promptly. |
| *A~B: A or more and less than B | | | |

[Table 2]

| Moisture Decrease Rate (%) ("Amount Of Dewater replacement (g)/Initial Body Weight (g)" * 100) | Main Phenomena | Level Display | Warning Display |
|---|---|---|---|
| less than 1% | Not applicable | Safe 1 | None |
| 1~2% | Degradation of performance starts, and motion becomes gradually slow. No subjective symptoms. | Safe 2 | Please promptly receive water replacement of • ml. |
| 2~3% | Feel thirsty, but have few subjective symptoms for degradation of performance. | Safe 3 | Please promptly receive water replacement of • ml. |
| 3~4% | Tiredness and painfulness come out, and degradation of performance becomes recognized by both oneself and others. | Caution | Please drop pace of exercise, and promptly receive water replacement of • ml. |
| 5% or more | Nausea, dizziness, and the like occur, and becomes danger state. | Danger | Please stop exercise, and promptly receive water replacement of • ml. |
| *A~B: A or more and less than B | | | |

[Display Device]

The calculation device 19 can select a level display and/or a warning display corresponding to the calculation result from the data stored in the storage device 18, and cause the display device 20 to display the level display and/or the warning display.

[Input Device]

The total body water content evaluation system can be provided with an input device having an input function of a measurement start instruction and a measurement cycle (min).

In a case where a CPU of a mobile terminal is used as the calculation device 19, a screen of the mobile terminal can be used as an input device 22.

The input device 22 can be used for inputting a measurement start instruction, a measurement cycle (min), body weight data, and a moisture intake amount.

<Circuit Configuration>

The first humidity sensor 7 and the second humidity sensor 8 of the perspiration meter 1 are respectively electrically connected to filter circuits F1, F2.

When the detection signals output from the respective sensors are input, the filter circuits F1 and F2 each remove a noise component included in each detection signal, and then amplify the detection signal at a prescribed amplification factor.

A differential amplifier DA1 is connected to outputs sides of the filter circuits F1, F2.

The output side of the filter circuit F1 is connected to an inverting input terminal (-) of the differential amplifier DA1.

The output side of the filter circuit F2 is connected to a non-inverting input terminal (+) of the differential amplifier DA1.

With the configuration, the differential amplifier DA1 outputs a signal of difference between a signal corresponding to the absolute humidity of the natural air detected by the first humidity sensor 7 and a signal corresponding to the absolute humidity of the mixed air detected by the second humidity sensor 8. The signal to be output from the differential amplifier DA1 corresponds to the local perspiration amount ($mg/cm^2/min$) that is the perspiration amount diffused from the skin surface to the mixture chamber 5.

When signals output from the differential amplifier DA1 are input to the calculation device 19, the calculation device 19 calculates a local perspiration amount ($mg/cm^2/min$) that is an actual perspiration amount diffused from the skin surface to the mixture chamber 5, on the basis of the respective input signals, and thereafter calculates a total body moisture loss amount per unit time (mg/min) from the formula (1).

In one embodiment, the calculation device 19 calculates a cumulated moisture loss amount (mg) subsequent to the measurement start instruction from the total body moisture loss amount per unit time (mg/min), the measurement cycle (min), and elapsed time after the measurement start instruction, calculates a water decrease rate (%) from the cumulated moisture loss amount (mg) and the body weight data, and selects a level display and/or a warning display corresponding to the water decrease rate (%) from the data stored in the storage device 18 and displays the level display and/or the warning display on the display device 20.

The system can also be configured to be reset by input of a moisture intake amount by a user with the input device 22 after the display.

In one embodiment, the user can also input, at an intake of water, the moisture intake amount and a measurement start instruction.

In this case, the calculation device 19 can also calculate a cumulated moisture loss amount (mg) subsequent to the measurement start instruction from the total body moisture loss amount per unit time (mg/min), the measurement cycle (min), and elapsed time after the measurement start instruction, and display to encourage water supply on the display device 20 at a time point when the cumulated moisture loss amount (mg) exceeds the moisture intake amount.

In one embodiment, the calculation device 19 can also calculate an increase rate of the local perspiration amount ($mg/cm^2/min$), and display notice of an increase in the cumulated moisture loss amount (mg) on the display device 20 at a time point when the increase rate has remained high. Specifically, for example, the calculation device 19 calculates, for each predetermined time (for example, several tens of seconds to several minutes), a mean value ($X_i$) of local perspiration amounts in a relevant section, and performs the display at a time point when an increase rate ($dX_i=X_i-X_{i-1}$) from a mean value ($X_{i-1}$) of local perspiration amounts in a section immediately prior to the relevant section changes to decrease.

Generally, in exercising, perspiration starts with latent time (time from when the exercise is started to when the perspiration starts) of several minutes to several tens of minutes, the perspiration amount gently increases thereafter, and remains high at a certain value. When the perspiration amount remains high, because the cumulated moisture loss amount (mg) tends to suddenly increase thereafter, at a time point when the perspiration amount has remained high, an increase in the total body moisture loss amount (mg/min) is notified, and a notice display to encourage early water supply, whereby performance reduction in sporting can be prevented.

[Example]

A relation between a cumulated moisture loss amount measured by the total body water content evaluation system of the present invention and a body weight reduction amount was examined.

The cumulated moisture loss amount was calculated using the total body water content evaluation system of the device configuration in Fig. 1.

The opening portion 2 of the housing capsule 3 was attached to a wrist of a subject (male in 30's, body weight of 58 kg, body height of 165 cm), and step exercises for 10 minutes were repeated four times indoors (temperature: 25°C, humidity: 50%). The body weight was measured after the end of the step exercise each time. The body height and the body weight were used as personal information on the subject, and a body surface area was obtained therefrom. The subject wore a training wear in exercising, and took off the wear and wiped sweat, the body weight measurement was performed thereafter using a weight scale (BC-314 resolution of 50g made of TANITA corporation). No water supply was performed during the experiment.

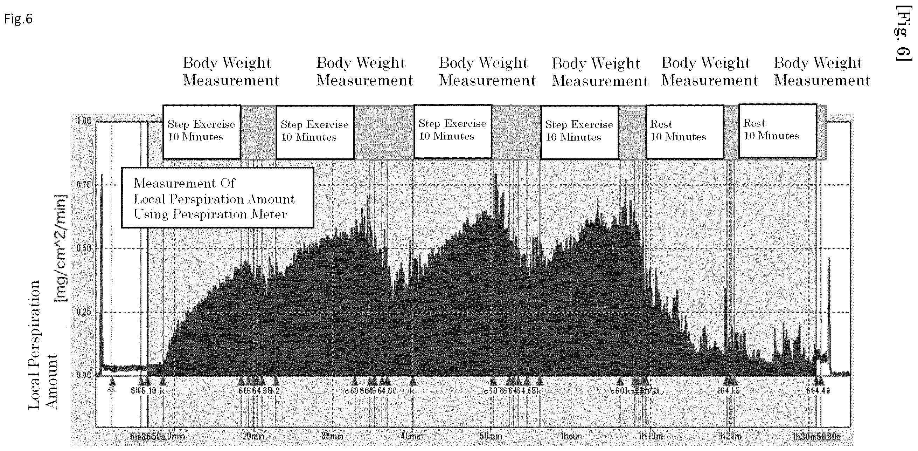

Fig. 6 illustrates a measurement result of the local perspiration amount ($mg/cm^2/min$) by the perspiration meter 1, and body weight measurement timing.

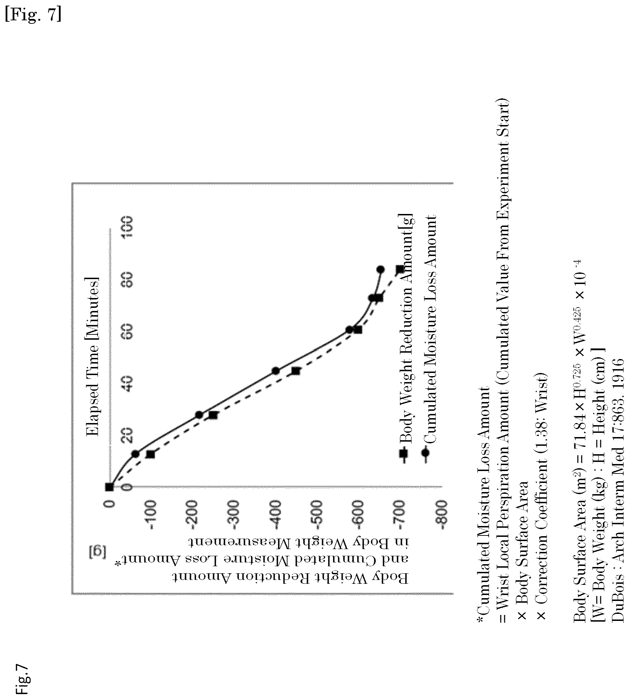

Fig. 7 illustrates comparison data between the cumulated moisture loss amount (g) subsequent to the measurement start instruction calculated by the total body water content evaluation system, and the body weight reduction amount (g).

As illustrated in Fig. 7, it was confirmed that the cumulated moisture loss amount (g) calculated by the total body water content evaluation system of the present invention indicated substantially the same value as the body weight reduction amount (g).

Industrial Applicability

For example, a perspiration meter configuring the total body water content evaluation system of the present invention is attached to a wrist or the like as a wearable form, and a system capable of processing measurement data by the perspiration meter with a smartphone application is constructed, whereby it is possible to grasp the moisture loss amount in real time during sports and in daily life, and encourage suitable water replacement.

Reference Signs List

| | |
|---|---|
| 1 | perspiration meter |
| 2 | opening portion |
| 3 | housing capsule |
| 4 | air suction hole |
| 5 | mixture chamber |
| 6 | air discharge hole |
| 7 | first humidity sensor |
| 8 | second humidity sensor |
| 13 | air blowing unit |
| 14 | flexible pipe |
| 15 | box |
| 16 | box |
| 17 | flexible pipe |
| 18 | storage device |
| 19 | calculation device |
| 20 | display device |
| 22 | input device |
| 30 | mobile terminal |
| 101A | sensor capable of detecting effective perspiration with high sensitivity |
| 101B | sensor capable of detecting invalid perspiration with high sensitivity |
| 102 | evaporation promoting mechanism |
| 103 | discharge unit |
| 104 | unit of summing perspiration amounts to be obtained from two or more types of sensors |
| 105 | moisture-proof mechanism |

**Claims**

1. A total body water content evaluation system comprising a calculation device that calculates a total body moisture loss amount per unit time from a local perspiration amount (mg/cm$^2$/min) obtained using a perspiration meter and a following formula (1).

$$\text{Total body moisture loss amount per unit time (mg/min)} = \text{local perspiration amount (mg/cm}^2\text{/min)} \times \text{correction coefficient for each measurement site} \times \text{body surface area (cm}^2) \ldots (1)$$

2. The total body water content evaluation system according to claim 1, wherein the perspiration meter is any one selected from a group consisting of following (A) to (D):

   (A) a perspiration meter including at least one or more types of sensors that measure a water content to be generated from a skin surface, and one or more evaporation promoting mechanisms that promote evaporation on the skin surface;
   (B) a perspiration meter including two or more types of sensors that measure the water content to be generated from the skin surface;
   (C) a perspiration meter including one or more types of sensors that measure the water content to be generated from the skin surface, a moisture-proof mechanism, and a discharge unit of a liquid; and
   (D) a perspiration meter including: a housing capsule including an opening portion to be attached to the skin surface, the housing capsule including an air suction hole for sucking natural air into an inside of the housing capsule, a mixture chamber that communicates with the opening portion to diffuse sweat on the skin surface, and in which the diffused sweat and the natural air are mixed and are made to be mixed air, and an air discharge hole for discharging the mixed air from the mixture chamber; a first humidity sensor for measuring humidity of the natural air; and a second humidity sensor for measuring humidity of the mixed air.

3. The total body water content evaluation system according to claim 1 or 2, further comprising an input device having an input function of a measurement start instruction and a measurement cycle (min), wherein
   the calculation device has a function of calculating a cumulated moisture loss amount (mg) subsequent to the measurement start instruction using the total body moisture loss amount per unit time (mg/min) and the measurement cycle (min).

4. The total body water content evaluation system according to claim 3, wherein

   the input device has a function of inputting body weight data, and
   the calculation device has a function of calculating a water decrease rate (%) from the cumulated moisture loss amount (mg) and the body weight data.

5. The total body water content evaluation system according to claim 4, further comprising a storage device that has stored therein a level display and/or a warning display in accordance with the water decrease rate (%).

6. The total body water content evaluation system according to claim 5, further comprising a display device that displays the level display and/or the warning display.

7. The total body water content evaluation system according to claim 6, wherein the calculation device selects a level display and/or a warning display in accordance with the water decrease rate (%) from the storage device, and displays the level display and/or the warning display on the display device.

8. The total body water content evaluation system according to claim 3, further comprising a display device that gives notice of an increase in the cumulated moisture loss amount (mg).

9. The total body water content evaluation system according to claim 8, wherein the calculation device calculates an increase rate of the local perspiration amount (mg/cm$^2$/min), and gives the notice when the increase rate decreases.

Fig.1

[Fig. 1]

EP 4 066 737 A1

12

[Fig. 2]

Fig.2

Fig.3A

Fig.3A

102

101A

Discharge Of Gas

1

Sensor
(Sensitive To Gas)

Enclosed Structure

Evaporation Promoting Mechanism
(Invalid Perspiration → Effective Perspiration)

Effective Perspiration

Invalid Perspiration

Skin

[Fig. 3A]

EP 4 066 737 A1

EP 4 066 737 A1

Fig.3B

102

Evaporation Promoting Mechanism
(Invalid Perspiration → Effective Perspiration)

1

Discharge Of Gas

101B

Effective Perspiration

Sensor
(Sensitive To Liquid)

Invalid Perspiration

Skin

[Fig. 4]

Fig.4

<u>1</u>

Unit Of Summing Sensor (1) + Sensor (2) — 104

Discharge Of Liquid

Discharge unit (Liquid) — 103

Enclosed Structure

Sensor(1) (Sensitive To Liquid) — 101B

Effective Perspiration

Invalid Perspiration

Skin

Sensor(2) (Sensitive To Gas) — 101A

Discharge Of Gas

Fig.5

105

Moisture-Proof Mechanism
(Effective Perspiration → Invalid Perspiration)

Discharge Of Liquid

Discharge unit (Liquid)

Effective Perspiration

Sensor
(Sensitive To Liquid)

Invalid Perspiration

Skin

103

101B

1

[Fig. 5]

EP 4 066 737 A1

EP 4 066 737 A1

[Fig. 6]

Fig.6

18

[Fig. 7]

Fig.7

*Cumulated Moisture Loss Amount
= Wrist Local Perspiration Amount (Cumulated Value From Experiment Start)
  × Body Surface Area
  × Correction Coefficient (1.38: Wrist)

Body Surface Area (m²) = $71.84 \times H^{0.725} \times W^{0.425} \times 10^{-4}$
[W= Body Weight (kg) : H = Height (cm) ]
DuBois : Arch Interm Med 17:863. 1916

## INTERNATIONAL SEARCH REPORT

| | International application No. |
|---|---|
| | PCT/JP2020/042618 |

| A. CLASSIFICATION OF SUBJECT MATTER |
|---|
| Int.Cl. A61B5/053(2021.01)i, A61B5/00(2006.01)i |
| FI: A61B5/00N, A61B5/05B |
| According to International Patent Classification (IPC) or to both national classification and IPC |

| B. FIELDS SEARCHED |
|---|
| Minimum documentation searched (classification system followed by classification symbols) |
| Int.Cl. A61B5/00, A61B5/053, A61B5/0537 |

| Documentation searched other than minimum documentation to the extent that such documents are included in the fields searched |  |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

| Electronic data base consulted during the international search (name of data base and, where practicable, search terms used) |
|---|
| JSTPlus/JMEDPlus/JST7580(JDreamIII) |

| C. DOCUMENTS CONSIDERED TO BE RELEVANT |
|---|

| Category* | Citation of document, with indication, where appropriate, of the relevant passages | Relevant to claim No. |
|---|---|---|
| Y<br>A | WO 2017/208650 A1 (SHARP CORPORATION) 07 December 2017 (2017-12-07)<br>paragraphs [0015], [0018]-[0023], [0038]-[0041], [0043]-[0046], fig. 1, 2<br>paragraphs [0015]-[0170], fig. 1-17 | 1-8<br>9 |
| Y<br>A | 坂口正雄，小野伸幸，中島浩二，菊池雅博，大橋俊夫，２チャネルデイジタル表示発汗計の応用 －ヒト各部位の発汗量計測－，信学技報，September 2000, MBE2000-57, pp. 31-36,「2.発汗量測定システムの概要」の欄,「3.発汗計解析ソフトウエア」の欄, fig. 1, entire text, all drawings, (SAKAGUCHI, Masao, ONO, Nobuyuki, NAKAJIMA, Koji, KIKUCHI, Masahiro, OHASHI, Toshio, IEICE Technical Report), non-official translation (Two channels digital display-type sweating ratemeter and it's applications: measurement of sweating amount of each part of human, "2. Summary of sweating amount measurement system", "3. Sweating ratemeter analysis software") | 1-8<br>9 |

☒ Further documents are listed in the continuation of Box C.　☒ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18 January 2021 | 02 February 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2020/042618 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 5-192301 A (SUZUKEN KK) 03 August 1993 (1993-08-03) paragraphs [0011]-[0013], fig. 1 | 1-8 |
| A | paragraphs [0011]-[0018], fig. 1-4 | 9 |
| A | JP 2010-46196 A (LIFE CARE GIKEN INC.) 04 March 2010 (2010-03-04), paragraphs [0014]-[0042], fig. 1-4 | 1-9 |

Form PCT/ISA/210 (second sheet) (January 2015)

INTERNATIONAL SEARCH REPORT
Information on patent family members

International application No.

PCT/JP2020/042618

| | | | |
|---|---|---|---|
| WO 2017/208650 A1 | 07 December 2017 | US 2019/0290186 A1 paragraphs [0031], [0034]-[0039], [0054]-[0057], [0059]-[0062], fig. 1, 2 | |
| JP 5-192301 A | 03 August 1993 | (Family: none) | |
| JP 2010-46196 A | 04 March 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6117101 B